# EUROPEAN PATENT SPECIFICATION

(11) **EP 0 808 172 B1**
(45) Date of publication and mention of the grant of the patent: **25.06.2003**
(21) Application number: 96910309.2
(22) Date of filing: 09.02.1996
(51) Int. Cl.: A61K 38/55, A61P 9/02

(54) **COMBINATION OF ANGIOTENSIN CONVERTING ENZYME INHIBITOR AND ALDOSTERONE ANTAGONIST FOR THE TREATMENT OF VENTRICULAR HYPERTROPHY**
KOMBINATION VON ACE-HEMMERN MIT ALDOSTERONANTAGONISTEN ZUR BEHANDLUNG DER VENTRIKULÄREN HYPERTROPHIE
COMBINAISON DE L'INHIBITEUR DE L'ENZYME DE CONVERSION DE L'ANGIOTENSINE ET D'UN ANTAGONISTE DE L'ALDOSTERONE POUR LE TRAITEMENT DE L'HYPERTROPHIE VENTRICULAIRE

(30) Priority: 10.02.1995 US 386852
(43) Date of publication of application: 26.11.1997
(60) Divisional application: 01116593.3
(73) Proprietor: G.D. SEARLE & COMPANY, Skokie Illinois 60077 (US)
(72) Inventor: MACLAUGHLAN, Todd, E., Grayslake, IL 60030 (US); PEREZ, Alfonso, T., Lake Forest, IL 60045 (US)
(74) Representative: Strych, Werner Dr.
(86) International application number: US9601969
(87) International publication number: WO96024373

(56) References cited:
- WO-A-95/09625
- US-A- 4 217 347
- EUROPEAN HEART JOURNAL, vol. 16, no. N, 1995, pages 107-110, XP000577583 B. PITT: "ACE INHIBITOR CO-THERAPY IN PATIENTS WITH HEART FAILURE: R.A.L.E.S."
- J. HEPATOLOGY, vol. 19, no. 2, 1993, IRELAND, pages 321-322, XP000577577 O.M.P. JOLOBE: "EFFICACY OF LOW-DOSE CAPTOPRIL IN ADDITION TO FUROSEMIDE AND SPIRONOLACTONE ... DURING BLUNTED DIURESIS"
- JOURNAL OF CARDIOVASCULAR PHARMACOLOGY, vol. 24, no. 2, 1994, pages 194-198, XP000577665 BERGLER-KLEIN ET AL: "SAFETY OF CONCOMITANT POTASSIUM-SPARING DIURETICS IN ACE-INHIBITOR THERAPY IN SEVERE CHF"
- THE AMERICAN JOURNAL OF CARDIOLOGY, vol. 65, no. 2, 1990, pages 33K-35K, XP000577438 PONCELET ET AL: "SPIRONOLACTONE AND ALTIZIDE USED IN COMBINATION WITH ELANAPRIL: 24-HOUR AMBULATORY RECORDING OF BLOOD PRESSURE" cited in the application
- THE NEW ENGLAND JOURNAL OF MEDICINE, vol. 316, no. 23, 1987, pages 1429-1435, XP000577598 SWEDBERG ET AL: "EFFECTS OF ENALAPRIL ON MORTALITY IN SEVERE CONGESTIVE HEART FAILURE"
- CHINESE MEDICAL JOURNAL, vol. 107, no. 9, 1994, pages 688-692, XP000577674 YA-LING ET AL: "COMBINED THERAPY OF CAPTOPRIL AND SPIRONOLACTONE FOR REFRACTORY CHF"
- CURRENT THERAPEUTICAL RESEARCH, CLIN. EXP., vol. 51, no. 2, 1992, pages 235-248, XP000577676 DAHLSTROM, KARLSSON: "CAPTOPRIL AND SPIRONOLACTONE THERAPY IN PATIENTS WITH REFRACTORY CHF"
- PROCESS IN PHARMACOLOGY AND CLINICAL PHARMACOLOGY, vol. 10, no. 3, 1995, pages 33-45, XP000577659 B.J. MATERSON: "DIURETIC THERAPY OF HYPERTENSION IN THE US: LOW DOSE BECOMES LOWER"
- JOURNAL OF CARDIOVASCULAR PHARMACOLOGY, vol. 22, no. SUP6, 1993, pages S1-S7, XP000577597 H. KNAUF: "THE ROLE OF LOW-DOSE DIURETICS IN ESSENTIAL HYPERTENSION"

## Description

### Field of the Invention

Combinations of an angiotensin converting enzyme inhibitor and an aldosterone receptor antagonist are described for use in treatment of circulatory disorders, including cardiovascular diseases such as heart failure, hypertension and congestive heart failure. Of particular interest are therapies using a spirolactone-type aldosterone receptor antagonist compound in combination with an angiotensin converting enzyme inhibitor, using a side-effect-reduced amount of the aldosterone receptor antagonist.

### Background of the Invention

Myocardial (or cardiac) failure, that is, heart failure ("HF"), whether a consequence of previous myocardial infarction(s), heart disease associated with hypertension, or primary cardiomyopathy, is a major health problem of worldwide proportions. The incidence of symptomatic heart failure has risen steadily over the past several decades.

In clinical terms, decompensated cardiac failure consists of a constellation of signs and symptoms that arise from congested organs and hypoperfused tissues to form congestive heart failure (CHF) syndrome. Congestion is caused largely by increased venous pressure and by inadequate sodium (Na⁺) excretion, relative to dietary Na⁺ intake, and is importantly related to circulating levels of aldosterone (ALDO). An abnormal retention of Na⁺ occurs via tubular epithelial cells throughout the nephron, including the later portion of the distal tubule and cortical collecting ducts, where ALDO receptor sites are present.

ALDO is the body's most potent mineralocorticoid hormone. As connoted by the term mineralocorticoid, this steroid hormone has mineral-regulating activity. It promotes Na⁺ reabsorption not only in the kidney, but also from the lower gastrointestinal tract and salivary and sweat glands, each of which represents classic ALDO-responsive tissues. ALDO regulates Na⁺ and water resorption at the expense of potassium (K⁺) and magnesium (Mg²⁺) excretion.

ALDO can also provoke responses in non-epithelial cells. Elicited by a chronic elevation in plasma ALDO level that is inappropriate relative to dietary Na⁺ intake, these responses can have adverse consequences on the structure of the cardiovascular system. Hence, ALDO can contribute to the progressive nature of myocardial failure for multiple reasons.

Multiple factors regulate ALDO synthesis and metabolism, many of which are operative in the patient with myocardial failure. These include renin as well as non-renin-dependent factors (such as K⁺, ACTH) that promote ALDO synthesis. Hepatic blood flow, by regulating the clearance of circulating ALDO, helps determine ALDO plasma concentration, an important factor in heart failure characterized by reduction in cardiac output and hepatic blood flow.

The renin-angiotensin-aldosterone system ("RAAS") is one of the hormonal mechanisms involved in regulating pressure/volume homeostasis and also in the development of hypertension, a precursor condition implicated in the progression of more serious cardiovascular diseases such as congestive heart failure. Activation of the renin-angiotensin-aldosterone system begins with secretion of the enzyme renin from the juxtaglomerular cells in the kidney. The enzyme renin acts on a naturally-occurring substrate, angiotensinogen, to release a decapeptide, Angiotensin I. This decapeptide is cleaved by angiotensin converting enzyme ("ACE") to provide an octapeptide, Angiotensin II, the primary active species of this system. This octapeptide, angiotensin II, is a potent vasoconstrictor and also produces other physiological effects such as stimulating aldosterone secretion, promoting sodium and fluid retention, inhibiting renin secretion, increasing sympathetic nervous system activity, stimulating vasopressin secretion, causing positive cardiac inotropic effect and modulating other hormonal systems.

Emphasis has been placed on minimizing hyperaldosteronism as a basis for optimizing patient treatment. This includes the importance of ALDO-receptor antagonism both in patients treated with conventional diuretic programs and in patients treated with angiotensin-converting enzyme (ACE) inhibitors, who are often constrained to small doses of ACE inhibitor because of orthostatic hypotension. Such patients may demonstrate a recurrence of heart failure symptoms likely related to elevations in plasma ALDO levels.

Many aldosterone receptor blocking drugs and their effects in humans are known. For example, spironolactone is a drug which acts at the mineralocorticoid receptor level by competitively inhibiting aldosterone binding. This steroidal compound has been used for blocking aldosterone-dependent sodium transport in the distal tubule of the kidney in order to reduce edema and to treat essential hypertension and primary hyperaldosteronism [F. Mantero et al, Clin. Sci. Mol. Med., 45 (Suppl 1), 219s-224s (1973)]. Spironolactone is also used commonly in the treatment of other hyperaldosterone-related diseases such as liver cirrhosis and congestive heart failure [F.J. Saunders et al, Aldactone; Spironolactone: A Comprehensive Review, Searle, New York (1978)]. Progressively-increasing doses of spironolactone from 1 mg to 400 mg per day [i.e., 1 mg/day, 5 mg/day, 20 mg/day] was administered to a spironolactone-intolerant patient to treat cirrhosis-related ascites [P.A. Greenberger et al, N. Eng. Reg. Allergy Proc., 7(4), 343-345 (Jul-Aug, 1986)]. It has been recognized that development of myocardial fibrosis is sensitive to circulating levels of both Angiotensin II and aldosterone, and that the aldosterone antagonist spironolactone prevents myocardial fibrosis in animal models, thereby linking aldosterone to excessive collagen deposition [D. Klug et al, Am. J. Cardiol., 71(3), 46A-54A (1993)]. Spironolactone has been shown to prevent fibrosis in animal models irrespective of the development of left ventricular hypertrophy and the presence of hypertension [C.G. Brilla et al, J. Mol. Cell. Cardiol., 25(5), 563-575 (1993)]. Spironolactone at a dosage ranging from 25 mg to 100 mg daily is used to treat diuretic-induced hypokalemia, when orally-administered potassium supplements or other potassium-sparing regimens are considered inappropriate [Physicians' Desk Reference, 46th Edn., p. 2153, Medical Economics Company Inc., Montvale, N.J. (1992)].

Previous studies have shown that inhibiting ACE inhibits the renin-angiotensin system by substantially complete blockade of the formation of Angiotensin II. Many ACE inhibitors have been used clinically to control hypertension. While ACE inhibitors may effectively control hypertension, side effects are common including chronic cough, skin rash, loss of taste sense, proteinuria and neutropenia.

Moreover, although ACE inhibitors effectively block the formation of Angiotensin II, aldosterone levels are not well controlled in certain patients having cardiovascular diseases. For example, despite continued ACE inhibition in hypertensive patients receiving captopril, there has been observed a gradual return of plasma aldosterone to baseline levels [J. Staessen et al, J. Endocrinol., 91, 457-465 (1981)]. A similar effect has been observed for patients with myocardial infarction receiving zofenopril [C. Borghi et al, J. Clin. Pharmacol., 33, 40-45 (1993)]. This phenomenon has been termed "aldosterone escape".

Combinations of an aldosterone antagonist and an ACE inhibitor have been investigated for treatment of heart failure. It is known that mortality is higher in patients with elevated levels of plasma aldosterone and that aldosterone levels increase as CHF progresses from RAAS activation. Routine use of a diuretic may further elevate aldosterone levels. ACE inhibitors consistently inhibit angiotensin II production but exert only a mild and transient antialdosterone effect.

Combining an ACE inhibitor and spironolactone has been suggested to provide substantial inhibition of the entire RAAS. For example, a combination of enalapril and a 25 mg daily dose of spironolactone has been administered to ambulatory patients with monitoring of blood pressure [P. Poncelet et al, Am. J. Cardiol., 65(2), 33K-35K (1990)]. In a 90-patient study, a combination of spironolactone at a dose in a range from 50mg/day to 100 mg/day (average 73 mg/day) and captopril was administered and found effective to control refractory CHF without serious incidents of hyperkalemia [U. Dahlstrom et al, Am. J. Cardiol., 71, 29A-33A (21 Jan 1993)]. Spironolactone dosage at 100 mg/day coadministered with an ACE inhibitor was reported to be highly effective in 13 of 16 patients afflicted with congestive heart failure, with a 25 mg/day to 50 mg/day spironolactone maintenance dosage given at trial completion to compensated patients being treated with an ACE inhibitor and loop diuretic [A.A. van Vliet et al, Am. J. Cardiol., 71, 21A-28A (21 Jan 1993)]. Clinical improvements have been reported for patients receiving a co-therapy of spircnolactone and the ACE inhibitor enalapril, although this report mentions that controlled trials are needed to determine the lowest effective doses and to identify which patients would benefit most from combined therapy [F. Zannad, Am. J. Cardiol., 71(3), 34A-39A (1993)].
J. Hepatology, vol 19, No. 2, 1996, pp 321-322 discloses the effect of captopril to furosemide/spironolacton during blunted diuresis.
J. Cardiovascular Pharmacology, vol 24, No. 2, 1994, pp 194-198 describes the safety of potassium -sparing diuretics in ACE- inhibitor therapy in severe CHF.
Amer.J. Cardiology, vol 65, No. 2, 1990 pp 33K- 35K is concerned with the use of spironolactone and altizide together with elanapril for an 24 hour ambulatory recording of blood pressure.
The Chinese Medical Journal, vol 107, No. 9, 1997, pp 688-692 describes the combined therapy of captopril and spironolactone for refractory CHF.
In the "Current Therapeutical Research", Chin. Exp., vol 51, No.2, 1992, pp 235- 2 also the captopril/spironolactone therapy in refractory CHF is discussed.

US-A 4217 347 is also concerned with the use of captopril/spironolactone combination for treating hypertension.

### Summary of Drawing Figures

Fig. 1 shows urinary aldosterone levels at different rates of spironolactone administration (12.5 mg, 25 mg, 50mg, 75mg), as compared to placebo, co-administered with stable doses of ACE inhibitor and loop diuretic.

Fig. 2 shows plasma renin activity at different rates of spironolactone administration (12.5 mg, 25 mg, 50mg, 75mg), as compared to placebo, co-administered with stable doses of ACE inhibitor and loop diuretic.

Fig. 3 shows N-Terminal ANT levels at different rates of spironolactone administration (12.5 mg, 25mg, 50mg, 75mg), as compared to placebo, co-administered with stable doses of ACE inhibitor and loop diuretic.

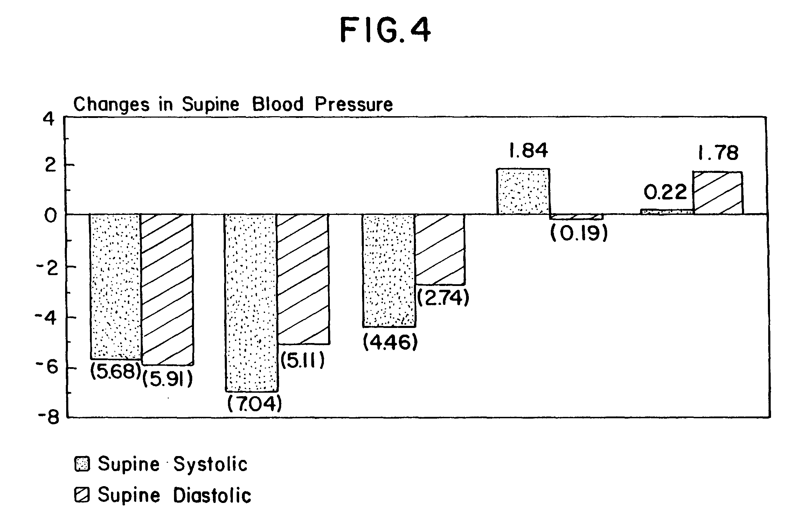

Fig. 4 shows changes in supine blood pressure at different rates of spironolactone administration (12.5 mg, 25mg, 50mg, 75mg), as compared to placebo, co-administered with stable doses of ACE inhibitor and loop diuretic.

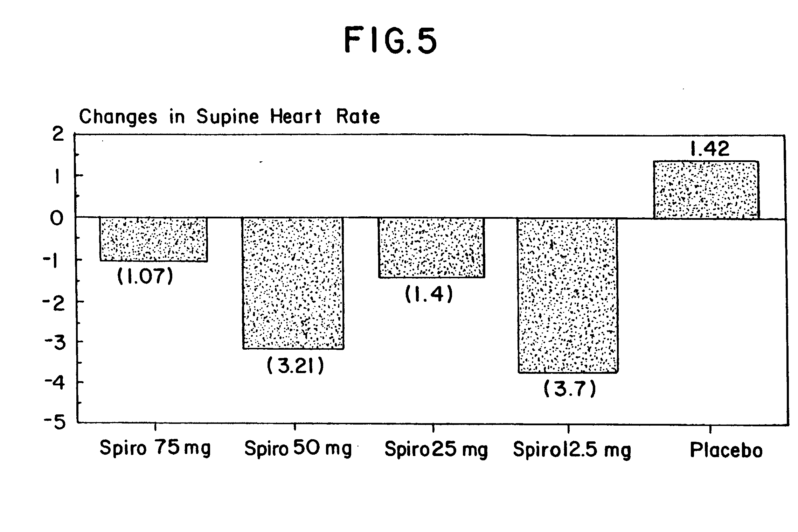

Fig. 5 shows changes in supine heart rate at different rates of spironolactone administration(12.5 mg, 25mg, 50mg, 75mg), as compared to placebo co-administered with stable doses of ACE inhibitor and loop diuretic.

### Description of the invention

Treatment or prevention of ventricular hypertrophy, is provided by a combination therapy comprising a therapeutically- effective amount of an angiotensin converting enzyme ("ACE") inhibitor along with a therpeutically effective amount of a spironolactone- type aldosterone receptor antagonist.
The spironolactone- type aldosteron receptor antagonist is present in such a combination and used in the therapy at a low dose, that is, at a dose lower than has been conventionally used in clinical situations. Such a low dose means a range from 1 mg + 25mg per dose.

The combination therapy of the invention would be useful, for example, to prevent or retard, in a subject, the development of ventricular hypertrophy.

Such subject would not typically be suffering from an edematous condition and thus would not gain benefit from treatment with conventional diuretic therapy as with loop diuretics which can alter electrolyte balance and cause hypokalemic or hypomagnesia conditions.

The phrase "angiotensin converting enzyme inhibitor" ("ACE inhibitor") is intended to embrace an agent or compound, or a combination of two or more agents or compounds, having the ability to block, partially or completely, the rapid enzymatic conversion of the physiologically inactive decapeptide form of angiotensin ("Angiotensin I") to the vasoconstrictive ociapeptide form of angiotensin ("Angiotensin II"). Blocking the formation of Angiotensin II can quickly affect the regulation of fluid and electrolyte balance, blood pressure and blood volume, by removing the primary actions of Angiotensin II. Included in these primary actions of Angiotensin II are stimulation of the synthesis and secretion of aldosterone by the adrenal cortex and raising blood pressure by direct constriction of the smooth muscle of the arterioles.

The phrase "aldosterone receptor antagonist" embraces an agent or compound, or a combination of two or more of such agents or compounds, which agent or compound binds to the aldosterone receptor as a competitive inhibitor of the action of aldosterone itself at an aldosterone receptor site, such as typically found in the renal tubules, so as to modulate the receptor-mediated activity of aldosterone. Typical of such aldosterone receptor antagonists are spirolactone-type compounds. The term "spirolactone-type" is intended to characterize a steroidal structure comprising a lactone moiety attached to a steroid nucleus, typically at the steroid "D" ring, through a spiro bond configuration.

The phrase "combination therapy" (or "co-therapy"), in defining use of an ACE inhibitor agent and an aldosterone receptor antagonist agent, is intended to embrace administration of each agent in a sequential manner in a regimen that will provide beneficial effects of the drug combination, and is intended as well to embrace coadministration of these agents in a substantially simultaneous manner, such as in a single capsule having a fixed ratio of these active agents or in multiple, separate capsules for each agent.

The phrase "therapeutically-effective" is intended to qualify the amount of each agent for use in the combination therapy which will achieve the goal of improvement in cardiac sufficiency by reducing or preventing, for example, the progression of congestive heart failure, while avoiding adverse side effects typically associated with each agent.

The phrase "low-dose amount", in characterizing a therapeutically-effective amount of the aldosterone receptor antagonist agent in the combination therapy, is intended to define a quantity of such agent, or a range of quantity of such agent, that is capable of improving cardiac sufficiency while reducing or avoiding one or more aldosterone-antagonist-induced side effects, such as hyperkalemia. A dosage of spironolactone which would accomplish the therapic goal of favorably enhancing the disorder mentioned above, while reducing or avoiding side effects, would be a dosage that substantially avoids inducing diuresis, that is, a substantially non-diuresis-effective dosage. Such doses is in the range from 1mg to 25 mg per dose.

A preferred combination therapy would consist essentially of two active agents, namely, an ACE inhibitor agent and aldosterone receptor antagonist agent. The agents would be used in combination in a weight ratio range from about 0.5-to-one to about twenty-to-one of the angiotensin converting enzyme agent to the aldosterone receptor antagonist agent. A preferred range of these two agents (ACE inhibitor-to-ALDO antagonist) would be from about one-to-one to about fifteen-to-one, while a more preferred range would be from about one-to-one to about five-to-one, depending ultimately on the selection of the ACE inhibitor and ALDO antagonist.

Examples of ACE inhibitors which may be used in the combination therapy are shown in the following four categories.

A first group of ACE inhibitors consists of the following compounds: AB-103, ancovenin, benazeprilat, BRL-36378, BW-A575C, CGS-13928C, CL-242817, CV-5975, Equaten, EU-4865, EU-4867, EU-5476, foroxymithine, FPL 66564, FR-900456, Hoe-065, I5B2, indolapril, ketomethylureas, KRI-1177, KRI-1230, L-681176, libenzapril, MCD, MDL-27088, MDL-27467A, moveltipril, MS-41, nicotianamine, pentopril, phenacein, pivopril, rentiapril, RG-5975, RG-6134, RG-6207, RGH-0399, ROO-911, RS-10085-197, RS-2039, RS 5139, RS 86127, RU-44403, S-8308, SA-291, spiraprilat, SQ-26900, SQ-28084, SQ-28370, SQ-28940, SQ-31440, Synecor, utibapril, WF-10129, Wy-44221, Wy-44655, Y-23785, Yissum P-0154, zabicipril and.

A second group of ACE inhibitors of interest consists of the following compounds: Asahi Brewery AB-47, alatriopril, BMS 182657, Asahi Chemical C-111, Asahi Chemical C-112, Dainippon DU-1777, mixanpril, Prentyl, zofenoprilat and 1-(-(1-carboxy-6-(4-piperidinyl)hexyl) amino)-1-oxopropyl octahydro-1H-indole-2-carboxylic acid.

A third group of ACE inhibitors of greater interest consists of the following compounds: Bioproject BP1.137, Chiesi CHF 1514, Fisons FPL-66564, idrapril, Marion Merrell Dow MDL-100240, perindoprilat and Servier S-5590.

A fourth group of ACE inhibitors of highest interest consists of the following compounds: alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat and spirapril.

Many of these ACE inhibitors are commercially available, especially those listed in the fourth group, above. For example, a highly preferred ACE inhibitor, captopril, is sold by E.R. Squibb & Sons, Inc., Princeton, N.J., now part of Bristol-Myers-Squibb, under the trademark "CAPOTEN", in tablet dosage form at doses of 12.5 mg, 50 mg and 100 mg per tablet. Enalapril or Enalapril Maleate, and Lisinopril are two more highly preferred ACE inhibitors sold by Merck & Co, West Point, Pa. Enalapril is sold under the trademark "VASOTEC" in tablet dosage form at doses of 2.5 mg, 5 mg, 10 mg and 20 mg per tablet. Lisinopril is sold under the trademark "PRINIVIL" in tablet dosage form at doses of 5 mg, 10 mg, 20 mg and 40 mg per tablet.

A family of spirolactone-type compounds of interest is defined by Formula I 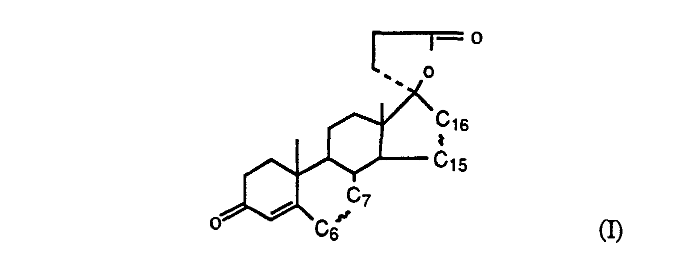 wherein 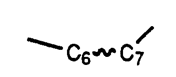 is 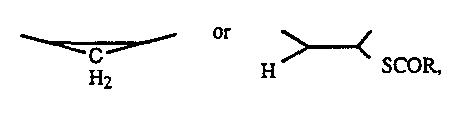 wherein R is lower alkyl of up to 5 carbon atoms, and
wherein 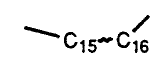 is

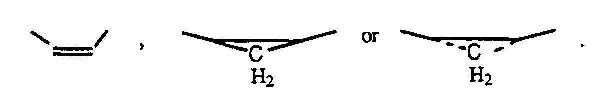

Lower alkyl residues include branched and unbranched groups, preferably methyl, ethyl and n-propyl.

Specific compounds of interest within Formula I are the following:
7α-Acetylthio-3-oxo-4,15-androstadiene-[17(β-1')-spiro-5']perhydrofuran-2'-one;
3-Oxo-7α-propionylthio-4,15-androstadiene-[17((β-1')-spiro-5']perhydrofuran-2'-one;
6β,7β-Methylene-3-oxo4,15-androstadiene-[17((β-1')-spiro-5']perhydrofuran-2'-one;
15α,16α-Methylene-3-oxo-4,7a-propionylthio-4-androstene[17(β-1')-spiro-5']perhydrofuran-2'-one;
6β,7β,15α,16α-Dimethylene-3-oxo-4-androstene [17(β-1')-spiro-5']perhydrofuran-2'-one;
7α-Acetylthio-15β,16β-Methylene-3-oxo-4-androstene-[17(β-1')-spiro-5']perhydrofuran-2'-one;
15β,16β-Methylene-3-oxo-7β-propionylthio-4-androstene-[17(β-1')-spiro-5']perhydrofuran-2'-one; and
6β,7β,15β,16β-Dimethylene-3-oxo-4-androstene-[17(β-1')-spiro-5']perhydrofuran-2'-one.

Methods to make compounds of Formula I are described in U.S. Patent No. 4,129,564 to Wiechart et al issued on 12 December 1978.

A second family of spirolactone-type compounds of interest is defined by Formula II: 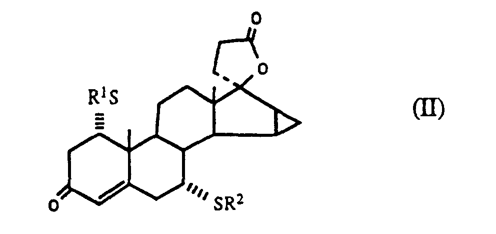 wherein R¹ is C₁₋₃-alkyl or C₁₋₃ acyl and R² is H or C₁₋₃-alkyl.

Specific compounds of interest within Formula II are the following:
1α-Acetylthio-15β,16β-methylene-7α-methylthio-3-oxo-17α-pregn-4-ene-21,17-carbolactone; and
15β,16β-Methylene-1α,7α-dimethylthio-3-oxo-17α-pregn-4-ene-21,17-carbolactone.

Methods to make the compounds of Formula II are described in U.S. Patent No. 4,789,668 to Nickisch et al which issued 6 December 1988.

A third family of spirolactone-type compounds of interest is defined by a structure of Formula III: 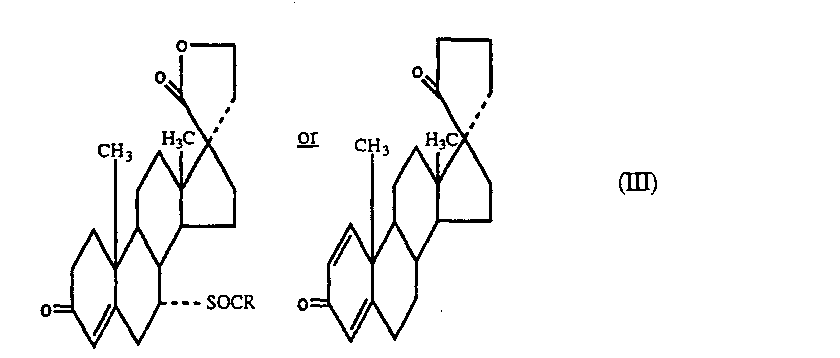 wherein R is lower alkyl, with preferred lower alkyl groups being methyl, ethyl, propyl and butyl. Specific compounds of interest include:
3β,21-dihydroxy-17α-pregna-5,15-diene-17-carboxylic acid γ-lactone;
3β,21-dihydroxy-17α-pregna-5,15-diene-17-carboxylic acid γ-lactone 3-acetate;
3β,21-dihydroxy-17α-pregn-5-ene-17-carboxylic acid γ-lactone;
3β,21-dihydroxy-17a-pregn-5-ene-17-carboxylic acid γ-lactone 3-acetate;
21-hydroxy-3-oxo-17α-pregn-4-ene-17-carboxylic acid γ-lactone;
21-hydroxy-3-oxo-17α-pregna-4,6-diene-17-carboxylic acid γ-lactone;
21-hydroxy-3-oxo-17α-pregna-1,4-diene-17-carboxylic acid γ-lactone;
7α-acylthio-21-hydroxy-3-oxo-17α-pregn-4-ene-17-carboxylic acid γ-lactones; and
7α-acetylthio-21-hydroxy-3-oxo-17α-pregn-4-ene-17-carboxylic acid γ-lactone.

Methods to make the compounds of Formula III are described in U.S. Patent No. 3,257,390 to Patchett which issued 21 June 1966.

A fourth family of compounds of interest is represented by Formula IV: 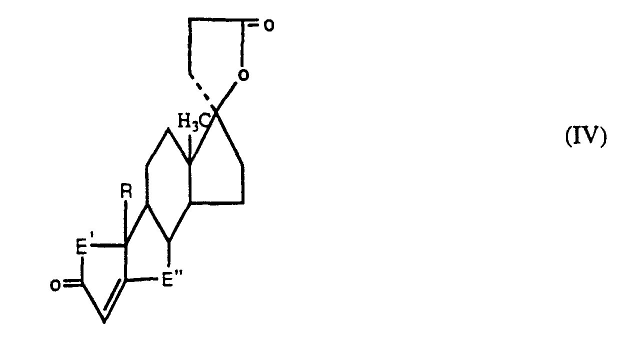 wherein E' is selected from the group consisting of ethylene, vinylene and (lower alkanoyl)thioethylene radicals, E" is selected from the group consisting of ethylene, vinylene, (lower alkanoyl)thioethylene and (lower alkanoyl)thiopropylene radicals; R is a methyl radical except when E' and E" are ethylene and (lower alkanoyl) thioethylene radicals, respectively, in which case R is selected from the group consisting of hydrogen and methyl radicals; and the selection of E' and E" is such that at least one (lower alkanoyl)thio radical is present.

A preferred family of compounds within Formula IV is represented by Formula V:

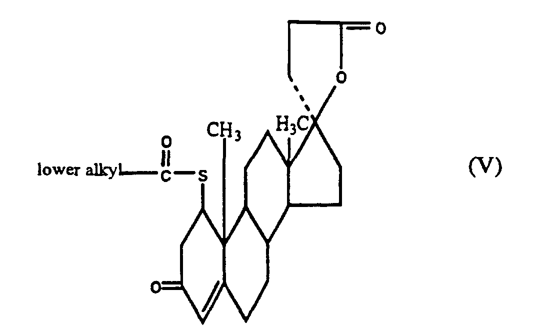

A more preferred compound of Formula V is 1-acetylthio-17α-(2-carboxyethyl)-17β-hydroxy-androst-4-en-3-one lactone.

Another preferred family of compounds within Formula IV is represented by Formula VI:

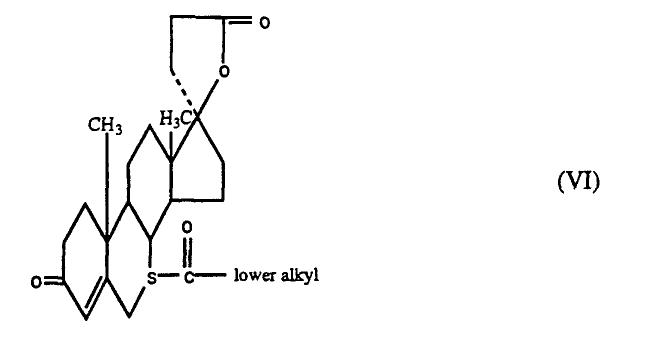

More preferred compounds within Formula VI include the following:
7α-acetylthio-17α-(2-carboxyethyl)-17β-hydroxyandrost-4-en-3-one lactone;
7β-acetylthio-17α-(2-carboxyethyl)-17β-hydroxyandrost-4-en-3-one lactone;
1α,7α-diacetylthio-17α-(2-carboxyethyl)-17β-hydroxy-androsta-4,6-dien-3-one lactone;
7α-acetylthio-17α-(2-carboxyethyl)-17β-hydroxyandrosta-1,4-dien-3-one lactone;
7α-acetylthio-17α-(2-carboxyethyl)-17β-hydroxy-19-norandrost-4-en-3-one lactone; and
7α-acetylthio-17α-(2-carboxyethyl)-17β-hydroxy-6α-methylandrost-4-en-3-one lactone;

In Formula IV-VI, the term "alkyl" is intended to embrace linear and branched alkyl radicals containing one to about eight carbons. The term "(lower alkanoyl)thio" embraces radicals of the formula lower alkyl

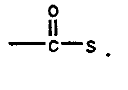

Of particular interest is the compound spironolactone having the following structure and formal name: 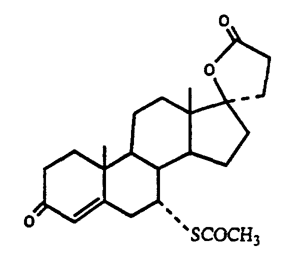 "spironolactone": 17-hydroxy-7α-mercapto-3-oxo-17α-pregn-4-ene-21-carboxylic acid γ-lactone acetate

Methods to make compounds of Formula IV-VI are described in U.S. Patent No. 3,013,012 to Cella et al which issued 12 December 1961. Spironolactone is sold by G.D. Searle & Co., Skokie, Illinois, under the trademark "ALDACTONE", in tablet dosage form at doses of 25 mg, 50 mg and 100 mg per tablet.

A diuretic agent may be used with the combination of ACE inhibitor and aldosterone receptor antagonist. Such diuretic agent may be selected from several known classes, such as thiazides and related sulfonamides, potassium-sparing diuretics, loop diuretics and organic mercurial diuretics.

Examples of thiazides are bendroflumethiazide, benzthiazide, chlorothiazide, cyclothiazide, hydrochlorotthiazide, hydroflumethiazode, methyclothiazide, polythiazide and trichlormethiazide.

Examples of related sulfonamides are chlorthalidone, quinethazone and metolazone.

An example of a non-thiazide sulfonamide diuretic is metolazone.

Examples of potassium-sparing diuretics are triameterene and amiloride.

Examples of loop diuretics, i.e., diuretics acting in the ascending limb of the loop of Henle of the kidney, are furosemide and ethynacrylic acid.

Examples of organic mercurial diuretics are mercaptomerin sodium, merethoxylline procaine and mersalyl with theophylline.

### Biological Evaluation

### Human Clinical Trials

A combination therapy of ACE inhibitor and spironolactone was evaluated in humans as described in the following clinical trials.

Patients: Two-hundred fourteen (214) patients with symptomatic heart failure had an ejection fraction ≤35%, a history of New York Heart Association (NYHA) functional classification III-IV six months prior to enrollment, and current classification II-IV were randomized among five treatment groups. Patients were assigned to receive either spironolactone 12.5 mg (41 patients), 25 mg (45 patients), 50 mg (47 patients), 75 mg (41 patients), or placebo (40 patients) once a day for 12 weeks. Two patients that were randomized failed to take the study medication and were excluded from the analysis. All patients were taking a stable dose of ACE inhibitor, loop diuretic, and optional digitalis for 30 days prior to the first dose of study medication. Potassium supplement therapy that was stable for 14 days prior to the first dose of study medication was also allowed. Informed consent was obtained from all patients, and the protocol was approved by each ethical committee. At enrollment all patients had normal serum potassium values (<5.5 mmol/L) and creatinine values of ≤2.0 mg/dL or ≤180 mmol/L. Patients were excluded from enrollment if they: (1) were diagnosed with either an acute life-threatening disease (included patients with automatic implantable cardioverter/ defibrillator), valvular disease, unstable angina, insulin-dependent diabetes, cancer (without a reoccurrence within the last five years), or primary hepatic failure; (2) were on a waiting list for a heart transplant or experienced a myocardial infarction 30 days prior to the first dose of study medication; (3) had laboratory values for hematology or biochemistry considered abnormal and clinically significant prior to the first dose of study medication; (4) received a potassium spacing diuretic within 30 days prior to the first dose of study medication.; (5) were receiving, on a regular basis, either non-steroidal antiinflammatory drugs or aspirin >325 mg/day, steroids, dopamine agonists or antagonists, insulin or heparin; (6) were on any investigational medication within 30 days of the first dose of medication.

Study Design: This was a multinational, double-blind, randomized, parallel group study.

Laboratory Measurements: The following information was obtained from each patient at baseline:
1. Concurrent medication within the past 30 days.
2. 12-lead ECG
3. Cardiac assessments that included blood pressure, pulse, sodium retention score (general assessment of a patient's edematous state was derived from the summation of scores obtained from Table I), NYHA classification, and
4. Signs and symptoms within the past 30 days.

**Table I**

| Sodium Retention Score | | |
|---|---|---|
| Parameters | Grade | Assessment |
| Rales | 0 | Absent |
| | 1 | In lower 1/3 of lungs |
| | 2 | In lower 2/3 of lungs |
| | 3 | In all lung fields |
| Peripheral | 0 | Absent |
| Pitting Edema | 1 | Trace |
| | 2 | Limited to ankles |
| | 3 | Not limited to ankles |
| | 4 | Anasarca |
| Weight Change | -1 | Decreased |
| | 0 | Unchanged |
| | 1 | Increased |
| Hepatomegaly | 0 | Absent |
| | 1 | Present |
| S3 Gallop | 0 | Absent |
| | 1 | Present |
| Increased Jugular | 0 | Absent |
| Venous Pressure | 1 | Present |

The following laboratory values were obtained at the pretreatment visit:

| | |
|---|---|
| Hematology | White blood cell count (WBC), hematocrit, hemoglobin, platelet count. |
| Biochemistry | Creatinine, potassium, AST, SGOT, urinary sodium/ potassium ratio, bicarbonate, calcium, chloride, creatinine, creatinine clearance, magnesium, glucose, urea, uric acid. |
| Neurohormones | Plasma renin activity, pro-atrial natriuretic factor, urinary aldosterone. |

Blood and urine samples were centrally analyzed at SciCor Laboratories. Laboratory values for urinary aldosterone and renin levels were done at the Ohio State University Laboratory in Columbus, Ohio. Pro-atrial natriuretic factor samples were evaluated at the University of Oslo Laboratory in Oslo, Norway. Patients were evaluated 9 days after beginning study medication. Documented changes in concurrent medications, signs and symptoms and drug compliance were recorded. These procedures were repeated at Week 4 and Week 8 visits. Patient information and procedures on the final visit (Week 12) was identical to the pre-treatment visit.

**Statistical Analysis:** Analysis of cardiac assessment changes in patient therapy and vital signs were performed for both the Intent-to-Treat (ITT) and evaluable patient groups. Analysis of demographic variables, adverse events and clinical laboratory values were performed in the ITT group. For each efficacy variable, results of each visit were examined separately. An appropriate trend test was used to test for overall dose-response. Pair-wise comparisons were made for each active dose to placebo. Significant levels for pair-wise comparisons were adjusted using the Hochberg-Bonferromi method to maintain the overall Type I error rate. All statistical methods were two-sided.

**Recruitment**: Two-hundred and fourteen patients were recruited from 22 study sites in eleven countries.

**Patient Characteristics**: Patient demographic, vital signs, and cardiac status at baseline are summarized in Table II.

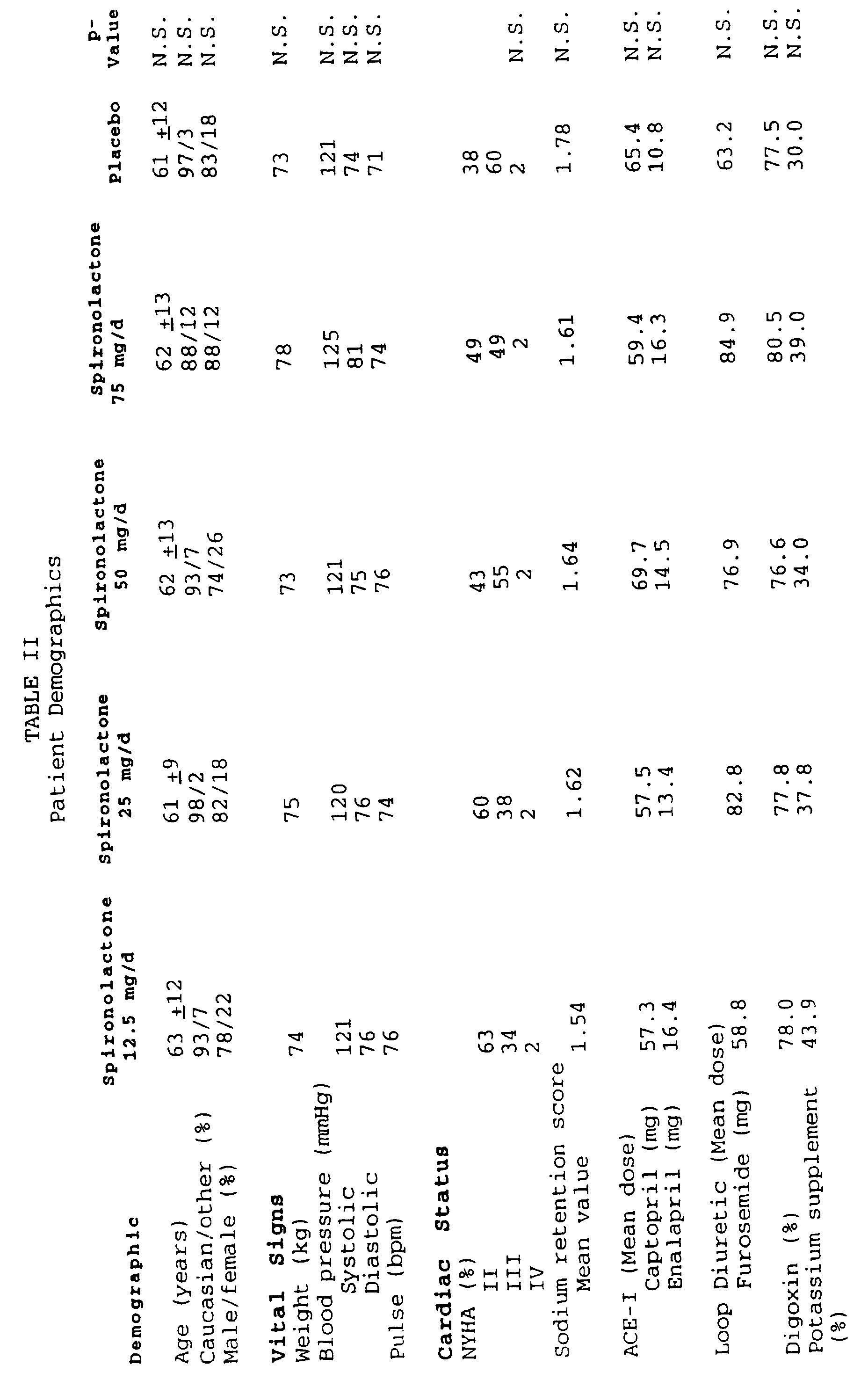

Patients ranged in age from 26 to 83 years (mean = 60), 81% were male, 94% were Caucasian. At baseline 51% of the patients were NYHA Class II, 47% were Class III. With respect to sodium retention score, a statistically significant dose response was seen at Day 9 with higher doses showing more reduction in sodium retention score (p = 0.019). However, this effect was not seen at later visits (p >0.20). There was an improvement in NYHA Class placebo group and in all the spironolactone groups. Although a trend toward improvement in the spironolactone group was observed, the difference was not statistically significant.

**Changes in Patient Therapy:** The treatment groups did not differ significantly with respect to changes in dose of ACE inhibitor, digitalis or potassium supplements at any visit (p ≥0.11). The treatment groups did differ significantly with respect to changes in loop diuretic therapy only at Week 8 (p = 0.004) in that more patients on the higher doses of spironolactone had decreases in the loop diuretic dose compared to the placebo group. This pattern was not observed at Week 12.

**Changes in Vital Signs:** Changes from baseline in vital signs at Week 12 are summarized in Table III.

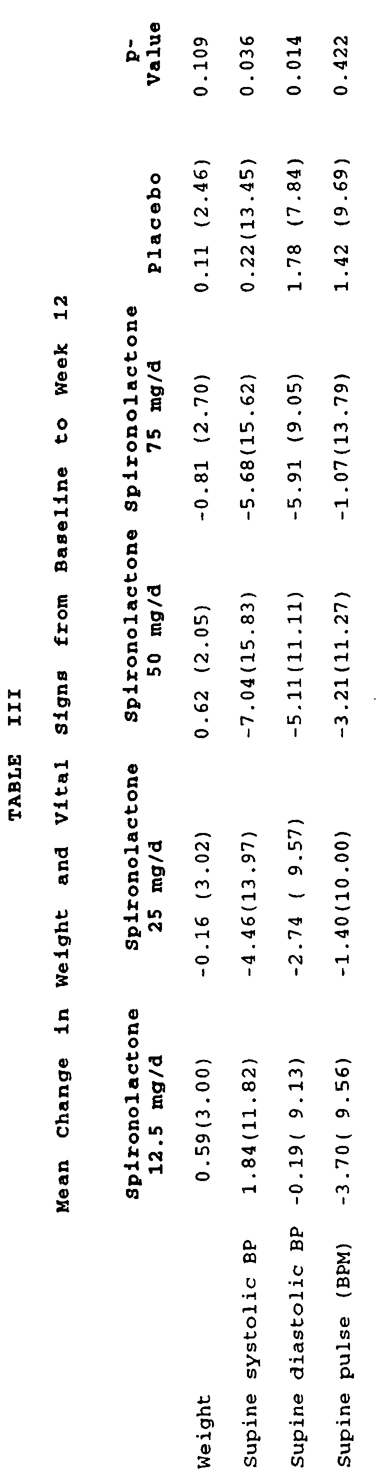

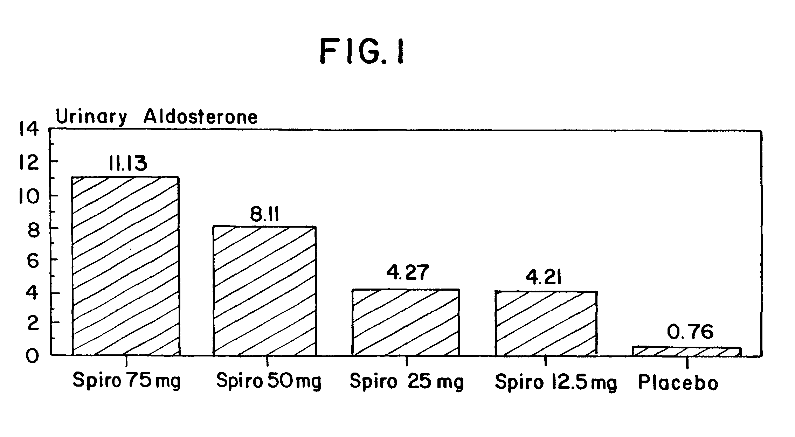

At all visits the 25 mg, 50 mg, and 75 mg groups had decreases in mean systolic and diastolic blood pressure, while the placebo group had increases in mean systolic and diastolic blood pressure (both standing and supine). Dose response with respect to standing and supine diastolic blood pressure was statistically significant for all visits (p ≤0.002). Dose response with respect to standing and supine blood pressure was statistically significant at Week 4, Week 8, and Week 12 (p ≤0.033), but not at Day 9 (p ≥0.12). No significant between-treatment differences in change from baseline in pulse were observed at any visit (p-values ≥0.136). A statistically significant dose response with greater decreases in pulse in the supine position at higher doses was observed at Week 4 (p-value = 0.045). Spironolactone doses of 25 and 50 mg were also significantly different from placebo (p-values ≤0.043) (See Fig. 1). At Day 9 and Week 4 visits, there was a statistically significant dose response with respect to changes from baseline in body weight in that patients in the 75 mg dose group experienced more weight loss than other patients. This dose response was not observed at later visits (p ≥0.062).

**Clinical Laboratory Values:** Table IV contains details of the different clinical laboratory values that showed statistically significant treatment differences with respect to mean changes at Week 12 visit compared with their respective baseline value.

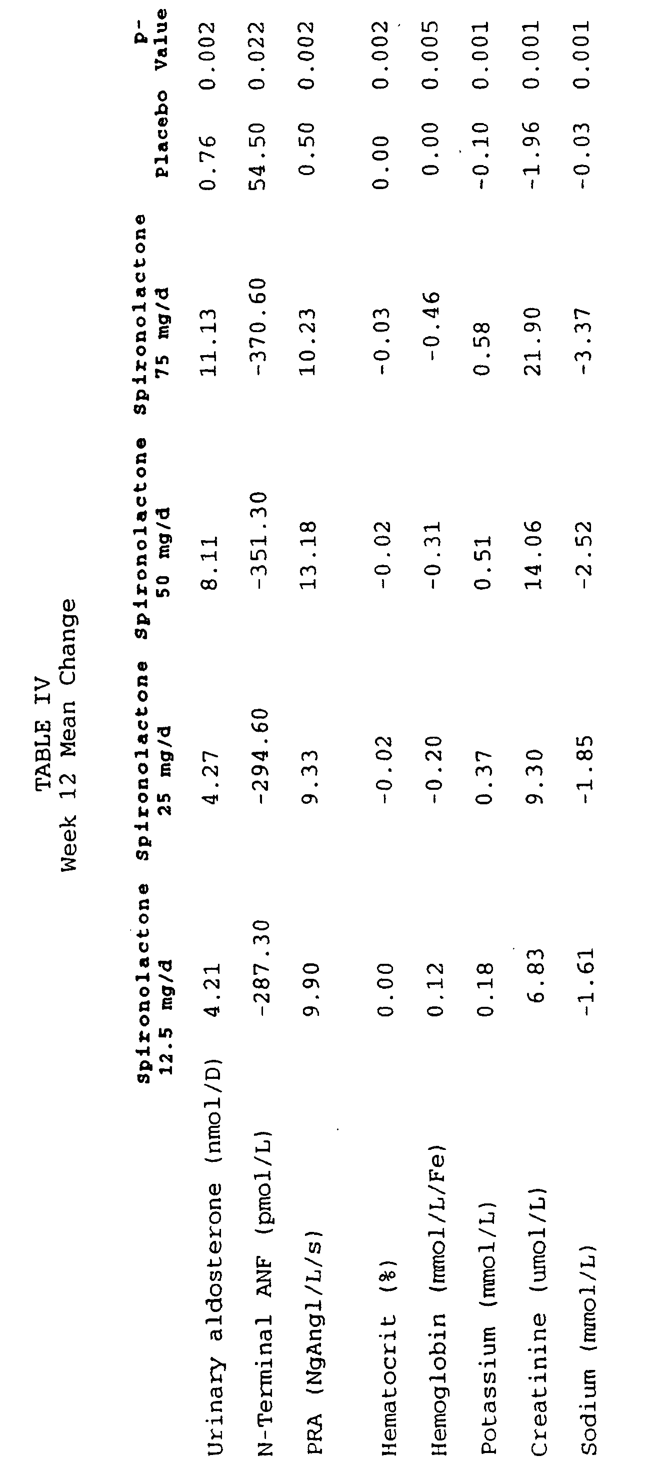

**Urinary Aldosterone (See Fig. 1):** Urinary aldosterone was determined only for baseline and the 12 week visit. Urinary aldosterone excretion showed mean increases from baseline in all treatment groups (P ≤0.012). Greater increases were seen at higher doses of spironolactone (p = 0.002). All pair-wise comparisons between active treatment and placebo were statistically significant (p ≤0.009).

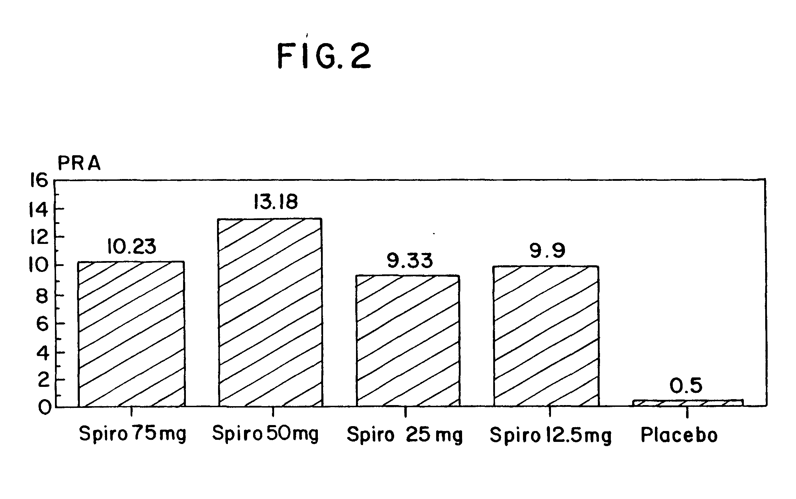

**Plasma Renin Activity (PRA) (See Fig. 2):** A statistically significant dose-response with respect to change from baseline in PRA was seen at Day 9, Week 4 and Week 12 (P ≤0.001) with higher doses of spironolactone associated with greater increases in PRA. PRA was not measured at Week 8.

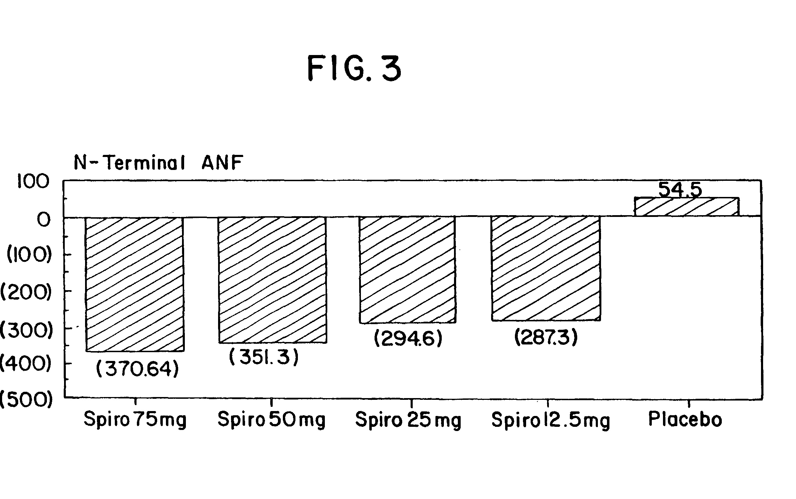

**N-Terminal Atrial Natriuretic Factor (ANF) (See Fig. 3):** All active treatments showed decreases from baseline at all treatment visits. Dose-response was statistically significant at Day 9 (p = 0.048), Week 4 (p = 0.005), and Week 12 (p = 0.008). ANF was not measured at Week 8. In comparisons the 50 mg dose group differed significantly from placebo at Week 4 (p = 0.009) and Week 12 (p = 0.006), while the 75 mg dose group differed significantly from placebo at Week 12 only (p = 0.007).

**Hematocrit and Hemoglobin:** At Day 9 visit a statistically significant mean value difference between placebo and the different active treatments was observed with lower values for the placebo group than the active treatments (p <0.001). At Week 12 a reverse statistically significant difference was observed with lower levels for the active treatment groups for hematocrit (p = 0.002) and hemoglobin (0.005).

**Serum Potassium**: A statistically significant dose-response with respect to change from baseline in serum potassium was seen at all treatment period visits (p <0.001). Higher doses of spironolactone were associated with larger increases in potassium. All doses of active treatment had significantly higher serum potassium levels relative to baseline than placebo (p ≤0.034)

### Incidence of Hyperkalemia

| | Spironolactone | Spironolactone | Spironolactone | Spironolactone |
|---|---|---|---|---|
| Treatment: Placebo | 12.5 mg/d | 25 mg/d | 50 mg/d | 75 mg/d |
| Patients (%)2(5%) | 2(5%) | 6(13%) | 9(20%) | 10(24%) |

**Predictors of Hyperkalemia:** Seven possible predictors of hyperkalemia (potassium ≥5.5 mmol/L) were included in a step-wise Cox regression analysis: randomized treatment (treated as a categorical variable), age, baseline NYHA class, baseline serum potassium, baseline PRA, baseline creatinine, baseline urinary aldosterone, and type and dose of ACE-I. Besides the dose of spironolactone, the following predictors of hyperkalemia were statistically significant in the step-wise regression analysis: type of ACE-I (captopril versus other), baseline serum creatinine, and baseline serum potassium. Results are summarized as follows:

| Factor | p-value | Risk Ratio |
|---|---|---|
| Captopril vs other ACE-I | 0.013 | 0.318 |
| Serum Creatinine > normal | 0.038 | 2.72 |
| Baseline Potassium > median | 0.040 | 2.32 |

In this analysis, the risk ratio can be thought of as the probability that the patient with the risk factor will develop hyperkalemia, relative to the probability that a patient without the risk factor will develop it. (For example, patients on captopril are about one-third as likely to develop hyperkalemia as a patient on another ACE-I.)

Risk ratios relative to placebo for the various doses of spironolactone are:

| Pose | p-value | Risk Ratio |
|---|---|---|
| Spironolactone 12.5 mg | 0.98 | 1.02 |
| Spironolactone 25 mg | 0.19 | 2.91 |
| Spironolactone 50 mg | 0.034 | 5.32 |
| Spironolactone 75 mg | 0.016 | 6.56 |

After adjusting for the above factors, other predictors included in the step-wise regression analysis were not significant (p-values ≥0.07). However, the following additional factor was significantly related to the development of hyperkalemia when considered apart from other predictors except the dose of spironolactone.

| Factor | p-value | Risk Ratio |
|---|---|---|
| High ACE-I Dose | 0.050 | 2.93 |

**Serum Magnesium:** Change from baseline in serum magnesium showed a statistically significant dose-response at Day 9 and Week 4 (p ≤0.048), with more patients in the placebo group showing decreases in serum magnesium. However, this effect was not seen at later visits (p ≥0.083).

**Adverse Effects:** Table V summarizes the twelve most common adverse events by different treatment groups. Only one symptom, hyperkalemia, showed a clear dose-response in term of incidence (p = 0.001).

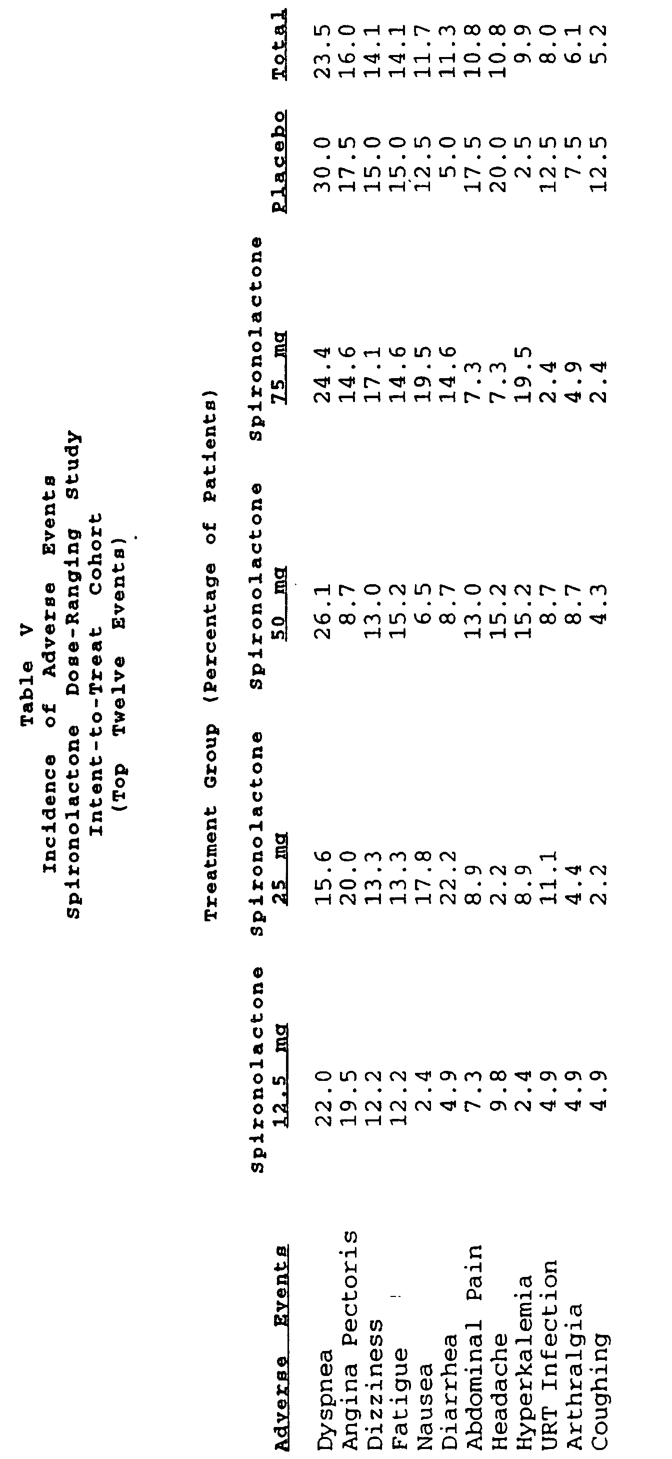

A breakdown of the hospitalizations is as follows:

| | | | | | | |
|---|---|---|---|---|---|---|
| Treatment | Placebo | 12.5 mg | 25 mg | 50 mg | 75 mg | P |
| Patients (%) | 5(12.5%) | 3(7.3%) | 3(6.6%) | 13(27.6%) | 6(14.6%) | |

No deaths were reported during the drug treatment period. Three patients died within 30 days after the study was completed. These three patients were previously at the 50 mg dose.

Administration of the angiotensin converting enzyme inhibitor and the aldosterone receptor antagonist may take place sequentially in separate formulations, or may be accomplished by simultaneous administration in a single formulation or separate formulations. Administration may be accomplished by oral route, or by intravenous, intramuscular or subcutaneous injections. The formulation may be in the form of a bolus, or in the form of aqueous or non-aqueous isotonic sterile injection solutions or suspensions. These solutions and suspensions may be prepared from sterile powders or granules having one or more pharmaceutically-acceptable carriers or diluents, or a binder such as gelatin or hydroxypropyl-methyl cellulose, together with one or more of a lubricant, preservative, surface-active or dispersing agent.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. The ACE inhibitor may be present in an amount from about 1 to 200 mg, preferably from about 2 to 150 mg, depending upon the specific ACE inhibitor selected. A suitable daily dose for a mammal may vary widely depending on the condition of the patient and other factors. The ALDO antagonist may be present in an amount of from about 1 to 400 mg, preferably from about 2 to 150 mg, depending upon the specific ALDO antagonist compound selected and the specific disease state being targeted for the combination therapy.

For disease states which require prevention, reduction or treatment of a cardiovascular disease state without incidence of hyperkalemia, for example, the ALDO antagonist component, typically spironolactone, will be present in the combination therapy in an amount in a range from about 1 mg to about 25 mg per dose. A preferred range for spirolactone would be from about 5 mg to 15 mg per dose. More preferably would be a range from about 10 mg to 15 mg per dose per day.

Examples of various fixed combinations of ACE inhibitor and ALDO antagonist representing a "double therapy" of the invention are as follow:

| ACE Inhibitor | | ALDO Antagonist |
|---|---|---|
| Captopril(mg)¹ | Enalapril (mg)² | Spironolactone (mg)² |
| 12.5 to 25 | 5 to 15 | 5 |
| 12.5 to 25 | 5 to 15 | 7.5 |
| 12.5 to 25 | 5 to 15 | 10 |
| 12.5 to 25 | 5 to 15 | 12.5 |
| 12.5 to 25 | 5 to 15 | 15 |
| 12.5 to 25 | 5 to 15 | 17.5 |
| 12.5 to 25 | 5 to 15 | 20 |
| 12.5 to 25 | 5 to 15 | 22.5 |

| | | |
|---|---|---|
| ¹Dose given 1, 2 or 3 times per day | | |
| ²Dose given once per day | | |

The active ingredients may also be administered by injection as a composition wherein, for example, saline, dextrose or water may be used as a suitable carrier.

The dosage regimen for treating a disease condition with the combination therapy of this invention is selected in accordance with a variety of factors, including the type, age, weight, sex and medical condition of the patient, the severity of the disease, the route of administration, and the particular compound employed, and thus may vary widely.

For therapeutic purposes, the active components of this combination therapy invention are ordinarily combined with one or more adjuvants appropriate to the indicated route of administration. If administered per os, the components may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The components may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Pharmaceutical compositions for use in the treatment methods of the invention may be administered in oral form or by intravenous administration. Oral administration of the combination therapy is preferred. Dosing for oral administration may be with a regimen calling for single daily dose, or for a single dose every other day, or for multiple, spaced doses throughout the day. The active agents which make up the combination therapy may be administered simultaneously, either in a combined dosage form or in separate dosage forms intended for substantially simultaneous oral administration. The active agents which make up the combination therapy may also be administered sequentially, with either active component being administered by a regimen calling for two-step ingestion. Thus, a regimen may call for sequential administration of the active agents with spaced-apart ingestion of the separate, active agents. The time period between the multiple ingestion steps may range from a few minutes to several hours, depending upon the properties of each active agent such a potency, solubility, bioavailability, plasma half-life and kinetic profile of the agent, as well as depending upon the age and condition of the patient. The active agents of the combined therapy whether administered simultaneously, substantially simultaneously, or sequentially, may involve a regimen calling for administration of one active agent by oral route and the other active agent by intravenous route. Whether the active agents of the combined therapy are administered by oral or intravenous route, separately or together, each such active agent will be contained in a suitable pharmaceutical formulation of pharmaceutically-acceptable excipients, diluents or other formulations components. Examples of suitable pharmaceutically-acceptable formulations containing the active components for oral administration are given below. Even though such formulations list both active agents together in the same recipe, it is appropriate for such recipe to be utilized for a formulation containing one of the active components.

### Example 1

An oral dosage may be prepared by screening and then mixing together the following list of ingredients in the amounts indicated. The dosage may then be placed in a hard gelatin capsule.

| Ingredients | Amounts |
|---|---|
| captopril | 62.0 mg |
| spironolactone | 12.5 mg |
| magnesium stearate | 10 mg |
| lactose | 100 mg |

### Example 2

An oral dosage may be prepared by mixing together granulating with a 10% gelatin solution. The wet granules are screened, dried, mixed with starch, talc and stearic acid, screened and compressed into a tablet.

| Ingredients | Amounts |
|---|---|
| captopril | 62.0 mg |
| spironolactone | 12.5 mg |
| calcium sulfate dihydrate | 100 mg |
| sucrose | 15 mg |
| starch | 8 mg |
| talc | 4 mg |
| stearic acid | 2 mg |

### Example 3

An oral dosage may be prepared by screening and then mixing together the following list of ingredients in the amounts indicated. The dosage may then be placed in a hard gelatin capsule.

| Ingredients | Amounts |
|---|---|
| enalapril | 14.3 mg |
| spironolactone | 12.5 mg |
| magnesium stearate | 10 mg |
| lactose | 100 mg |

### Example 4

An oral dosage may be prepared by mixing together granulating with a 10% gelatin solution. The wet granules are screened, dried, mixed with starch, talc and stearic acid, screened and compressed into a tablet.

| Ingredients | Amounts |
|---|---|
| enalapril | 14.3 mg |
| spironolactone | 12.5 mg |
| calcium sulfate dihydrate | 100 mg |
| sucrose | 15 mg |
| starch | 8 mg |
| talc | 4 mg |
| stearic acid | 2 mg |

## Claims

1. Use of a therapeutically-effective amount of an angiotensin converting enzyme inhibitor and an aldosterone receptor antagonist for preparing a medicament for treating a subject suffering from or susceptible to ventricular hypertrophy, wherein the aldosterone receptor antagonist is present in an amount that is therapeutically effective to antagonize aldosterone but insufficient to cause substantial diuretic effect, and wherein the aldosterone receptor antagonist is present in an amount in a daily dosage range from 1 mg to 25 mg per dose.

2. Use of claim 1 further **characterized by** administering said angiotensin converting enzyme inhibitor and said aldosterone receptor antagonist in a sequential manner.

3. Use of claim 1 further **characterized by** administering said angiotensin converting enzyme inhibitor and said aldosterone receptor antagonist in a substantially simultaneous manner.

4. Use of claim 1 wherein said aldosterone receptor antagonist is a spirolactone-type compound.

5. Use of claim 4 wherein said spirolactone-type compound is spirolactone.

6. Use of Claim 1 or 5 wherein said angiotensin converting enzyme inhibitor is selected from the group consisting of alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat, spirapril, Bioproject BP1.137, Chiesi CHF 1514, Fisons FPL-66564, idrapril, Marion Merrell Dow MDL-100240, perindoprilat, and Servier S-5590.

7. Use of claim 6 wherein said angiotensin converting enzyme inhibitor is selected from the group consisting of alacepril, benazepril, captopril, cilacapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat and spirapril.

8. Use of Claim 1 or 5 further **characterized by** said angiotensin converting enzyme inhibitor and said aldosterone receptor antagonist being used in said co-therapy in a weight ratio range from about 0.1-to-one to about twenty-five-to-one of said angiotensin converting enzyme inhibitor to said aldosterone receptor antagonist.

9. Use of claim 8 wherein said weight ratio range is from 0,5 - to - one to fifteen-to-one.

10. Use of claim 8 wherein said weight ratio range is from 0,5 - to - one to five -to-one.

11. Use according to any of Claims 6 to 10 wherein said angiotensin converting enzyme inhibitor is alacepril.

12. Use according to any of Claims 6 to 10 wherein said angiotensin converting enzyme inhibitor is benazepril.

13. Use according to any of Claims 6 to 10 wherein said angiotensin converting enzyme inhibitor is captopril.

14. Use according to any of Claims 6 to 10 wherein said angiotensin converting enzyme inhibitor is cilazapril.

15. Use according to any of Claims 6 to 10 wherein said angiotensin converting enzyme inhibitor is delapril.

16. Use according to any of Claims 6 to 10 wherein said angiotensin converting enzyme inhibitor is enalapril.

17. Use according to any of Claims 6 to 10 wherein said angiotensin converting enzyme inhibitor is enalaprilat.

18. Use according to any of Claims 6 to 10 wherein said angiotensin converting enzyme inhibitor is fosinopril.

19. Use according to any of Claims 6 to 10 wherein said angiotensin converting enzyme inhibitor is fosinoprilat.

20. Use according to any of Claims 6 to 10 wherein said angiotensin converting enzyme inhibitor is imidapril.

21. Use according to any of Claims 6 to 10 wherein said angiotensin converting enzyme inhibitor is lisinopril.

22. Use according to any of Claims 6 to 10 wherein said angiotensin converting enzyme inhibitor is perindopril.

23. Use according to any of Claims 6 to 10 wherein said angiotensin converting enzyme inhibitor is quinapril.

24. Use according to any of Claims 6 to 10 wherein said angiotensin converting enzyme inhibitor is ramipril.

25. Use according to any of Claims 6 to 10 wherein said angiotensin converting enzyme inhibitor is temocapril.

26. Use according to any of Claims 6 to 10 wherein said angiotensin converting enzyme inhibitor is trandolapril.

27. Use according to any of Claims 6 to 10 wherein said angiotensin converting enzyme inhibitor is ceranapril.

28. Use according to any of Claims 6 to 10 wherein said angiotensin converting enzyme inhibitor is moexipril.

29. Use according to any of Claims 6 to 10 wherein said angiotensin converting enzyme inhibitor is quinaprilat.

30. Use according to any of Claims 6 to 10 wherein said angiotensin converting enzyme inhibitor is spirapril.

31. Use according to any of Claims 6 to 10 wherein said angiotensin converting enzyme inhibitor is idrapril.

32. Use according to any of Claims 6 to 10 wherein said angiotensin converting enzyme inhibitor is perindoprilat.

33. Use of Claim 1 or 5 wherein said angiotensin converting enzyme inhibitor is captopril, in a daily dose range from about 30 mg to about 80 mg per dose, or is enalapril in a dose range from 5 to 25 mg per dose.

34. Use of claim 33 wherein said aldosterone receptor antagonist is spirolactone in a daily dose is in a range from 5mg to 20 mg.

35. Use of claim 34 wherein said spirolactone daily dose is in a range from 5mg to 20 mg.

36. Use of claim 34 wherein said spirolactone daily dose is in a range from 5mg to 15 mg.

## Patentansprüche

1. Verwendung einer therapeutisch wirksamen Menge eines Angiotensin-Converting-Enzyme-Hemmers (nachfolgend ACE-Hemmer) und eines Aldosteron-Rezeptor-Antagonisten zur Herstellung eines Arzneimittels zur Behandlung eines Patienten, der an ventrikulärer Hypertrophie leidet oder dafür empfänglich ist, worin der Aldosteron-Rezeptor-Antagonist in einer Menge vorliegt, die als Aldosteron-Antagonist wirksam, aber zur Verursachung einer wesentlichen diuretischen Wirkung ungenügend ist, und worin der Aldosteron-Rezeptor-Antagonist in einem Tagesdosisbereich in einer Menge zwischen 1 mg bis 25 mg pro Dosis vorliegt.

2. Verwendung nach Anspruch 1, weiter **gekennzeichnet durch** die nacheinander erfolgende Verabreichung des ACE-Hemmers und des Aldostreron-Rezeptor-Antagonisten.

3. Verwendung nach Anspruch 1, weiter **gekennzeichnet durch** die im wesentlichen gleichzeitig erfolgende Verabreichung des ACE-Hemmers und des Aldosteron-Rezeptor-Antagonisten.

4. Verwendung nach Anspruch 4, worin der Aldosteron-Rezeptor-Antagonist eine Verbindung vom Spirolacton-Typ ist.

5. Verwendung nach Anspruch 4, worin die Verbindung vom Spirolacton-Typ Spirolacton ist.

6. Verwendung nach Anspruch 1 oder 5, worin der ACE-Hemmer aus der Gruppe bestehend aus Alacepril, Benazepril, Captopril, Cilazapril, Delapril, Enalapril, Enalaprilat, Fosinopril, Fosinoprilat, Imidapril, Lisinopril, Perindopril, Quinapril, Ramipril, Saralasinacetat, Temocapril, Trandolapril, Ceranapril, Moexipril, Quinaprilat, Spirapril, Bioproject BP1.137 , Chiesi CHF 1514, Fisons FPL-66564, Idrapril, Marion Merrell Dow MDL-1 00240, Perindoprilat und Servier 5-5590 ausgewählt ist.

7. Verwendung nach Anspruch 6, worin der ACE-Hemmer aus der Gruppe bestehend aus Alacepril, Benazepril, Captopril, Cilacapril, Delapril, Enalapril, Enalaprilat, Fosinopril, Fosinoprilat, Imidapril, Lisinopril, Perindopril, Quinapril, Ramipril, Saralasinacetat, Temocapril, Trandolapril, Ceranapril, Moexipril, Quinaprilat und Spirapril ausgewählt ist.

8. Verwendung nach Anspruch 1 oder 5, weiter **gekennzeichnet durch** die cotherapeutische Verwendung des ACE-Hemmers und des Aldosteron-Rezeptor-Antagonisten in einem Gewichtsverhältnis im Bereich von etwa 0,1:1 bis etwa 25:1 ACE-Hemmer zu Aldosteron-Rezeptor-Antagonist.

9. Verwendung nach Anspruch 8, worin das Gewichtsverhältnis im Bereich von 0,5:1 bis 15:1 ist.

10. Verwendung nach Anspruch 8, worin das Gewichtsverhältnis im Bereich von 0,5:1 bis 5:1 ist.

11. Verwendung nach einem der Ansprüche 6 bis 10, worin der ACE-Hemmer Alacepril ist.

12. Verwendung nach einem der Ansprüche 6 bis 10, worin der ACE-Hemmer Benazepril ist.

13. Verwendung nach einem der Ansprüche 6 bis 10, worin der ACE-Hemmer Captopril ist.

14. Verwendung nach einem der Ansprüche 6 bis 10, worin der ACE-Hemmer Cilazapril ist.

15. Verwendung nach einem der Ansprüche 6 bis 10, worin der ACE-Hemmer Delapril ist.

16. Verwendung nach einem der Ansprüche 6 bis 10, worin der ACE-Hemmer Enalapril ist.

17. Verwendung nach einem der Ansprüche 6 bis 10, worin der ACE-Hemmer Enalaprilat ist.

18. Verwendung nach einem der Ansprüche 6 bis 10, worin der ACE-Hemmer Fosinopril ist.

19. Verwendung nach einem der Ansprüche 6 bis 10, worin der ACE-Hemmer Fosinoprilat ist.

20. Verwendung nach einem der Ansprüche 6 bis 10, worin der ACE-Hemmer Imidapril ist.

21. Verwendung nach einem der Ansprüche 6 bis 10, worin der ACE-Hemmer Lisinopril ist.

22. Verwendung nach einem der Ansprüche 6 bis 10, worin der ACE-Hemmer Perindopril ist.

23. Verwendung nach einem der Ansprüche 6 bis 10, worin der ACE-Hemmer Quinapril ist.

24. Verwendung nach einem der Ansprüche 6 bis 10, worin der ACE-Hemmer Ramipril ist.

25. Verwendung nach einem der Ansprüche 6 bis 10, worin der ACE-Hemmer Temocapril ist.

26. Verwendung nach einem der Ansprüche 6 bis 10, worin der ACE-Hemmer Trandolapril ist.

27. Verwendung nach einem der Ansprüche 6 bis 10, worin der ACE-Hemmer Ceranapril ist.

28. Verwendung nach einem der Ansprüche 6 bis 10, worin der ACE-Hemmer Moexipril ist.

29. Verwendung nach einem der Ansprüche 6 bis 10, worin der ACE-Hemmer Quinaprilat ist.

30. Verwendung nach einem der Ansprüche 6 bis 10, worin der ACE-Hemmer Spirapril ist.

31. Verwendung nach einem der Ansprüche 6 bis 10, worin der ACE-Hemmer Idrapril ist.

32. Verwendung nach einem der Ansprüche 6 bis 10, worin der ACE-Hemmer Perindoprilat ist.

33. Verwendung nach Anspruch 1 oder 5, worin der ACE-Hemmer Captopril in einem Tagesdosisbereich von etwa 30 mg bis etwa 80 mg pro Dosis oder Enalapril in einem Dosisbereich von 5 mg bis 25 mg pro Dosis ist.

34. Verwendung von Anspruch 33, worin der Aldosteron-Rezeptor-Antagonist Spirolacton in einem Tagesdosisbereich von 5 mg bis 20 mg ist.

35. Verwendung von Anspruch 34, worin der Spirolacton-Tagesdosisbereich von 5 mg bis 20 mg ist.

36. Verwendung von Anspruch 34, worin der Spirolacton-Tagesdosisbereich von 5 mg und 15 mg ist.

## Revendications

1. Utilisation d'une quantité thérapeutiquement efficace d'un inhibiteur d'enzyme de conversion de l'angiotensine et d'un antagoniste de récepteur de l'aldostérone pour préparer un médicament en vue de traiter un sujet souffrant ou susceptible d'hypertrophie ventriculaire, dans laquelle l'antagoniste de récepteur de l'aldostérone est présent selon une quantité thérapeutiquement efficace mais insuffisante pour provoquer un effet diurétique substantiel, et dans laquelle l'antagoniste de récepteur de l'aldostérone est présent selon une quantité dans un intervalle de dosage quotidien de 1 mg à 25 mg par dose.

2. Utilisation suivant la revendication 1, **caractérisée en outre par** l'administration dudit inhibiteur d'enzyme de conversion de l'angiotensine et dudit antagoniste de récepteur de l'aldostérone de façon séquencée.

3. Utilisation suivant la revendication 1, **caractérisée en outre par** l'administration dudit inhibiteur d'enzyme de conversion de l'angiotensine et dudit antagoniste de récepteur de l'aldostérone de façon substantiellement simultanée.

4. Utilisation suivant la revendication 1, dans laquelle ledit antagoniste de récepteur de l'aldostérone est un composé du type spirolactone.

5. Utilisation suivant la revendication 4, dans laquelle le composé du type spirolactone est la spirolactone.

6. Utilisation suivant la revendication 1 ou 5, dans laquelle ledit inhibiteur d'enzyme de conversion de l'angiotensine est choisi parmi l'ensemble constitué par les alacepril, benazepril, captopril, cilazapril, delapril, enalapril, enalaprilat, fosinopril, fosinoprilat, imidapril, lisinopril, perindopril, quinapril, ramipril, saralasin acétate, temocapril, trandolapril, ceranapril, moexipril, quinaprilat, spirapril, PB1.137 (de Bioproject), CHF 1514 (de Chiesi), FPL-66564 (de Fisons), idrapril, MDL-100240 (de Marion Merrell Dow), perindoprilat et S-5590 (de Servier).

7. Utilisation suivant la revendication 6, dans laquelle ledit inhibiteur d'enzyme de conversion de l'angiotensine est choisi parmi l'ensemble constitué par les alacepril, benazepril, captopril, cilazapril, delapril, enalapril, enalaprilat, fosinopril, fosinoprilat, imidapril, lisinopril, perindopril, quinapril, ramipril, saralasin acétate, temocapril, trandolapril, ceranapril, moexipril, quinaprilat et spirapril.

8. Utilisation suivant la revendication 1 ou 5, **caractérisée en outre par le fait que** ledit inhibiteur d'enzyme de conversion de l'angiotensine et ledit antagoniste de récepteur de l'aldostérone sont utilisés dans la co-thérapie selon un rapport pondéral inhibiteur d'enzyme de conversion de l'angiotensine/antagoniste de récepteur de l'aldostérone se situant dans l'intervalle de 0,1/1 à 25/1.

9. Utilisation suivant la revendication 8, dans laquelle ledit rapport pondéral se situe dans l'intervalle de 0,5/1 à 15/1.

10. Utilisation suivant la revendication 8, dans laquelle ledit rapport pondéral se situe dans l'intervalle de 0,5/1 à 5/1.

11. Utilisation suivant l'une quelconque des revendications 6 à 10, dans laquelle ledit inhibiteur d'enzyme de conversion de l'angiotensine est l'alacepril.

12. Utilisation suivant l'une quelconque des revendications 6 à 10, dans laquelle ledit inhibiteur d'enzyme de conversion de l'angiotensine est le benazepril.

13. Utilisation suivant l'une quelconque des revendications 6 à 10, dans laquelle ledit inhibiteur d'enzyme de conversion de l'angiotensine est le captopril.

14. Utilisation suivant l'une quelconque des revendications 6 à 10, dans laquelle ledit inhibiteur d'enzyme de conversion de l'angiotensine est le cilazapril.

15. Utilisation suivant l'une quelconque des revendications 6 à 10, dans laquelle ledit inhibiteur d'enzyme de conversion de l'angiotensine est le delapril.

16. Utilisation suivant l'une quelconque des revendications 6 à 10, dans laquelle ledit inhibiteur d'enzyme de conversion de l'angiotensine est l'enalapril.

17. Utilisation suivant l'une quelconque des revendications 6 à 10, dans laquelle ledit inhibiteur d'enzyme de conversion de l'angiotensine est l'enalaprilat.

18. Utilisation suivant l'une quelconque des revendications 6 à 10, dans laquelle ledit inhibiteur d'enzyme de conversion de l'angiotensine est le fosinopril.

19. Utilisation suivant l'une quelconque des revendications 6 à 10, dans laquelle ledit inhibiteur d'enzyme de conversion de l'angiotensine est le fosinoprilat.

20. Utilisation suivant l'une quelconque des revendications 6 à 10, dans laquelle ledit inhibiteur d'enzyme de conversion de l'angiotensine est l'imidapril.

21. Utilisation suivant l'une quelconque des revendications 6 à 10, dans laquelle ledit inhibiteur d'enzyme de conversion de l'angiotensine est le lisonopril.

22. Utilisation suivant l'une quelconque des revendications 6 à 10, dans laquelle ledit inhibiteur d'enzyme de conversion de l'angiotensine est le perindopril.

23. Utilisation suivant l'une quelconque des revendications 6 à 10, dans laquelle ledit inhibiteur d'enzyme de conversion de l'angiotensine est le quinapril.

24. Utilisation suivant l'une quelconque des revendications 6 à 10, dans laquelle ledit inhibiteur d'enzyme de conversion de l'angiotensine est le ramipril.

25. Utilisation suivant l'une quelconque des revendications 6 à 10, dans laquelle ledit inhibiteur d'enzyme de conversion de l'angiotensine est le temocapril.

26. Utilisation suivant l'une quelconque des revendications 6 à 10, dans laquelle ledit inhibiteur d'enzyme de conversion de l'angiotensine est le trandolapril.

27. Utilisation suivant l'une quelconque des revendications 6 à 10, dans laquelle ledit inhibiteur d'enzyme de conversion de l'angiotensine est le ceranapril.

28. Utilisation suivant l'une quelconque des revendications 6 à 10, dans laquelle ledit inhibiteur d'enzyme de conversion de l'angiotensine est le moexipril.

29. Utilisation suivant l'une quelconque des revendications 6 à 10, dans laquelle ledit inhibiteur d'enzyme de conversion de l'angiotensine est le quinaprilat.

30. Utilisation suivant l'une quelconque des revendications 6 à 10, dans laquelle ledit inhibiteur d'enzyme de conversion de l'angiotensine est le spirapril.

31. Utilisation suivant l'une quelconque des revendications 6 à 10, dans laquelle ledit inhibiteur d'enzyme de conversion de l'angiotensine est l'idrapril.

32. Utilisation suivant l'une quelconque des revendications 6 à 10, dans laquelle ledit inhibiteur d'enzyme de conversion de l'angiotensine est le perindoprilat.

33. Utilisation suivant la revendication 1 ou 5, dans laquelle ledit inhibiteur d'enzyme de conversion de l'angiotensine est le captopril, selon un dosage quotidien dans l'intervalle de 30mg à 80 mg par dose, ou l'enapril selon un dosage dans l'intervalle de 5 à 25 mg par dose.

34. Utilisation suivant la revendication 33, dans laquelle ledit antagoniste de récepteur de l'aldostérone est la spirolactone pour un dosage quotidien dans l'intervalle de 5 mg à 20 mg.

35. Utilisation suivant la revendication 34, dans laquelle ledit dosage quotidien en spirolactone se situe dans l'intervalle de 5 mg à 20 mg.

36. Utilisation suivant la revendication 34, dans laquelle ledit dosage quotidien en spirolactone se situe dans l'intervalle de 5 mg à 15 mg.
